# EUROPEAN PATENT APPLICATION

(11) **EP 4 454 699 A1**
(43) Date of publication of application: **30.10.2024**
(21) Application number: 22910105.0
(22) Date of filing: 21.12.2022
(51) Int. Cl.: A61N 1/36, A61N 1/05

(54) **NERVE STIMULATION APPARATUS AND SYSTEM, AND ELECTRONIC DEVICE AND STORAGE MEDIUM**

(30) Priority: 22.12.2021 CN 202111582620
(71) Applicant: Amygdala Neuro Technologies (Shenzhen) Co., Ltd., Shenzhen, Guangdong 518118 (CN)
(72) Inventor: MIN, Xiaoyi, Shenzhen, Guangdong 518118 (CN)
(74) Representative: Balder IP Law, S.L.
(86) International application number: PCT/CN2022/140842
(87) International publication number: WO 2023/116793

(57) **Abstract**

The present application relates to the field of medical instruments. Disclosed are a nerve stimulation apparatus and system, and an electronic device and a storage medium. The apparatus comprises: a pulse delivery module 201, a sensing module 202, and a control module 203, wherein the pulse delivery module 201 is used for delivering a pulse according to an instruction of the control module 203; the sensing module 202 is used for sensing an electrically evoked compound action potential according to an instruction of the control module 203; and the control module 203 is used for instructing, after pulse delivery of the current pulse delivery cycle, the sensing module 202 to sense an electrically evoked compound action potential, acquiring an ECAP peak-to-peak ratio of the electrically evoked compound action potential, and when the ECAP peak-to-peak ratio is not within a comfort interval, adjusting the amplitude of a subsequently delivered pulse until the ECAP peak-to-peak ratio is within the comfort interval, the comfort interval comprising a dimension of the amplitude of the pulse and a dimension of the ECAP peak-to-peak ratio of the electrically evoked compound action potential. Stress of different nerve fibers is reflected by using an ECAP peak-to-peak ratio, and the amplitude of a pulse is adjusted according to the ECAP peak-to-peak ratio, thereby improving the clinical effect of the nerve stimulation apparatus.

## Description

The present disclosure claims priority to Chinese Patent Application No. 202111582620.7, filed December 22, 2021, which is incorporated herein by reference in its entirety.

### TECHNICAL FIELD

Embodiments of the present disclosure relate in general to the field of medical instruments, and in particular to a nerve stimulation apparatus, a nerve stimulation system, an electronic device and a storage medium.

### BACKGROUND

With the progress and development of medical technology, the technology of controlling and treating diseases by sending stimulation pulses has gradually matured. Evoked compound action potential (ECAP) is a sum of all action potentials caused by electrical stimulation applied to nerve fibers. Clinical studies have demonstrated a large number of scientific evidence supporting the treatment of chronic pain via a closed-loop spinal cord electrical stimulation (SCS) system achieved on the basis of feedback control of ECAP. For example, the research founded by John Parker of using ECAP related parameters to characterize the effect of an amplitude of a spinal cord stimulation on dorsal column fiber activation has been widely studied. John Parker's research demonstrated that there is a functional relationship between a peak-to-peak value of ECAP and an amplitude of stimulation pulse, and the peak-to-peak value increases linearly with the increase of stimulation pulse after reaching a threshold. Moreover, ECAP is related to the patient's perception for stimulation pulses, and larger ECAP causes the patient to produce a stronger stimulation sensation. Recent clinical work has further demonstrated that stimulation frequency has an influence on the ECAP amplitude and perceived stimulation sensation of patients with chronic back and/or leg pain treated by the spinal cord electrical stimulation system.

The existing closed-loop spinal cord electrical stimulation system records the numerical value of the peak-to-peak value of ECAP generated in response to the stimulation pulses in the spinal cord after the stimulation pulses, compares the recorded peak-to-peak value of ECAP with a required target value or set value, and then automatically adjusts the generation of stimulation pulses in a next cycle according to the comparison result. The stimulation pulse can be adjusted according to the peak-to-peak value of ECAP, but the peak-to-peak value of ECAP cannot fully reflect the stress of nerve fibers since the peak-to-peak value of ECAP cannot reflect the action potentials caused by all nerve fibers with different diameters. Therefore, it's difficult to achieve the ideal clinical effect by only taking the peak-to-peak value of ECAP as the feedback basis of a spinal cord electrical stimulation system.

### SUMMARY

Embodiments of the present disclosure provide a nerve stimulation apparatus, a nerve stimulation system, an electronic device and a storage medium, which can achieve the closed-loop adjustment of the amplitude of the pulse to be generated in an efficient and simple way when it is determined that a peak-to-peak ratio of an evoked compound action potential triggered by the stimulation pulses is not within a comfort range, so that the patient can have a good feeling and treatment effect under the action of the stimulation pulses.

To achieve at least one of the above objects, some embodiments of the present disclosure provide a nerve stimulation apparatus including: a pulse generation module, a sensing module and a control module; where the pulse generation module is configured to generate pulses according to an instruction from the control module; the sensing module is configured to sense an evoked compound action potential according to an instruction from the control module; and the control module is configured to instruct the sensing module to sense the evoked compound action potential after a pulse is generated by the pulse generation module within a current pulse generation cycle, to obtain a peak-to-peak ratio of the evoked compound action potential, and to adjust, based on a determination that the peak-to-peak ratio of the evoked compound action potential is not within a comfort range, the amplitude of the pulse to be generated within a subsequent pulse generation cycle iteratively until the peak-to-peak ratio of the evoked compound action potential is within the comfort range; where the comfort range includes a dimension of amplitude of the pulse and a dimension of the peak-to-peak ratio of the evoked compound action potential.

To achieve at least one of the above objects, some embodiments of the present disclosure further provide a nerve stimulation system including: the nerve stimulation apparatus as described above and an electrode lead electrically connected with the nerve stimulation apparatus.

To achieve at least one of the above objects, some embodiments of the present disclosure further provide an electronic device including: at least one processor; and a memory communicatively connected to the at least one processor; wherein the memory stores instructions executable by the at least one processor, and the instructions, when executed by the at least one processor, cause the at least one processor to implement a method applied at a nerve stimulation apparatus. The control method includes: instructing to sense an evoked compound action potential after the pulse generation module generates a pulse within a current pulse generation cycle; obtaining a peak-to-peak ratio of the evoked compound action potential, and adjusting, based on a determination that the peak-to-peak ratio of the evoked compound action potential is not within a comfort range, the amplitude of the pulse to be generated by the pulse generation module within a subsequent pulse generation cycle iteratively until the peak-to-peak ratio of the evoked compound action potential is within the comfort range; where the comfort range includes a dimension of amplitude of the pulse and a dimension of peak-to-peak ratio of the evoked compound action potential.

To achieve at least one of the above objects, some embodiments of the present disclosure further provide a computer-readable storage medium storing a computer program. The computer program, when executed by a processor, causes the processor to implement a method applied at a nerve stimulation apparatus. The control method includes: instructing to sense an evoked compound action potential after the pulse generation module generates a pulse within a current pulse generation cycle; obtaining a peak-to-peak ratio of the evoked compound action potential, and adjusting, based on a determination that the peak-to-peak ratio of the evoked compound action potential is not within a comfort range, the amplitude of the pulse to be generated by the pulse generation module within a subsequent pulse generation cycle iteratively until the peak-to-peak ratio of the evoked compound action potential is within the comfort range; where the comfort range includes a dimension of amplitude of the pulse and a dimension of peak-to-peak ratio of the evoked compound action potential.

The nerve stimulation apparatus provided in the embodiments of the present disclosure senses an evoked compound action potential after a pulse is generated within a current pulse generation cycle, and adjusts the amplitude of the pulse to be generated within a subsequent pulse generation cycle if a peak-to-peak ratio of the evoked compound action potential is not within a comfort range, and obtains the new peak-to-peak ratio of the evoked compound action potential in the subsequent pulse generation cycle, and then determines whether the new peak-to-peak ratio of the evoked compound action potential is within the comfort range, and also adjusts, if the peak-to-peak ratio of the evoked compound action potential is still not within the comfort range, the amplitude of the pulse to be generated within the further subsequent pulse generation cycle iteratively until the obtained peak-to-peak ratio of the evoked compound action potential is within the comfort range. The nerve stimulation apparatus of the embodiments of the present disclosure takes the peak-to-peak ratio of the evoked compound action potential as the feedback parameter, which can reflect the action potentials caused by all nerve fibers with different diameters of the patient, fully reflecting the stress of nerve fibers. According to the present disclosure, making the action potential, caused by the finer nerve fibers that may easily cause discomfort in patients, to be within an appropriate range by controlling the amplitude of the pulse can provide more effective pulse therapy for patients as soon as possible, avoid discomfort of the patients caused by inappropriate pulse, and improve the clinical effect of the nerve stimulation apparatus.

### BRIEF DESCRIPTION OF THE DRAWINGS

One or more embodiments are illustrated exemplarily with reference to the accompanying drawings, and these exemplary descriptions do not constitute a limitation on the embodiments. Elements having the same reference numeral in the accompanying drawings indicate similar elements. The figures in the accompanying drawings do not constitute a scale limitation unless otherwise stated.
Fig. 1 is a schematic diagram of multiple ECAP curves under the action of stimulation pulses with the same frequency and different amplitudes;
Fig. 2 is a schematic structural diagram of a nerve stimulation apparatus according to an embodiment of the present disclosure;
Fig. 3 is a schematic diagram showing segments of the ECAP curves according to an embodiment of the present disclosure;
Fig. 4 is a schematic structural diagram of a nerve stimulation apparatus according to another embodiment of the present disclosure;
Fig. 5 is a schematic structural diagram of a nerve stimulation system according to another embodiment of the present disclosure; and
Fig. 6 is a schematic structural diagram of an electronic device according to another embodiment of the present disclosure.

### DETAILED DESCRIPTION OF THE EMBODIMENTS

In the conventional technology, the peak-to-peak value of ECAP generated in response to the stimulation pulses in the spinal cord is recorded after the stimulation pulses are generated. However, the stimulation pulse can be adjusted according to the peak-to-peak value of ECAP, but the peak-to-peak value of ECAP cannot fully reflect the stress of nerve fibers since the peak-to-peak value of ECAP cannot reflect the action potentials caused by all nerve fibers with different diameters. It's difficult to achieve the ideal clinical effect by only taking the peak-to-peak value of ECAP as the feedback basis of a spinal cord electrical stimulation system. Therefore, how to further improve the clinical effect of a nerve stimulation apparatus is an urgent problem to be solved.

In order to solve the above problem, some embodiments of the present disclosure provide a nerve stimulation apparatus including: a pulse generation module, a sensing module and a control module; where the pulse generation module is configured to generate pulses according to an instruction from the control module; the sensing module is configured to sense an evoked compound action potential according to an instruction from the control module; and the control module is configured to instruct the sensing module to sense the evoked compound action potential after a pulse is generated within a current pulse generation cycle, to obtain a peak-to-peak ratio of the evoked compound action potential, and to adjust, based on a determination that the peak-to-peak ratio of the evoked compound action potential is not within a comfort range, the amplitude of the pulse to be generated within a subsequent pulse generation cycle iteratively until the peak-to-peak ratio of the evoked compound action potential is within the comfort range; where the comfort range includes a dimension of amplitude of the pulse and a dimension of peak-to-peak ratio of the evoked compound action potential. The " peak-to-peak ratio of the evoked compound action potential" here refers to the ratio of the potential value of a second peak to the potential value of a third peak of the evoked compound action potential.

The nerve stimulation apparatus provided in the embodiments of the present disclosure senses an evoked compound action potential triggered by a pulse generated within a current pulse generation cycle to obtain the peak-to-peak ratio of the evoked compound action potential, and determines whether the amplitude of the pulse generated within the current pulse generation cycle is appropriate based on whether the peak-to-peak ratio of the evoked compound action potential is within a comfort range. Further, based on a determination that the peak-to-peak ratio of the evoked compound action potential is not within the comfort range, the amplitude of the pulse to be generated within a subsequent pulse generation cycle is adjusted iteratively until the obtained peak-to-peak ratio of the evoked compound action potential is within the comfort range. The nerve stimulation apparatus in the embodiments of the present disclosure can more comprehensively and accurately obtain the influence of the stimulation pulses on the excitability of nerve fibers with different diameters by using the peak-to-peak ratio of the evoked compound action potential as a parameter indicating the effectiveness of the pulse acting on the body. When the peak-to-peak ratio of the evoked compound action potential is not within the comfort range, the amplitude of the pulse is adjusted iteratively until the peak-to-peak ratio of the evoked compound action potential is within the comfort range, so that the action potential caused by the finer nerve fibers that may easily cause discomfort in patients is controlled to be within an appropriate range, avoid discomfort in patients, and improve the clinical effect of pulse therapy.

In order to make the objects, technical schemes and advantages of the embodiments of the present disclosure clearer, the embodiments of the present disclosure will be described in detail below with reference to the accompanying drawings. However, those of ordinary skill in the art may understand that in the embodiments of the present disclosure, many technical details are set forth in order to make the reader better understand the present disclosure. However, the technical solutions set forth in the present disclosure may still be implemented even without these technical details and various changes and modifications based on the following embodiments. The division of the following various embodiments is for convenience of description, which should not constitute any limitation on the specific implementations of the present disclosure, and various embodiments may be mutually referenced on the premise of no contradiction.

The implementation details of the nerve stimulation apparatus described in the present disclosure are specifically described below with reference to specific embodiments, and the following content merely describes implementation details for ease of understanding, and is not necessary for implementation of the technical solution in the present disclosure.

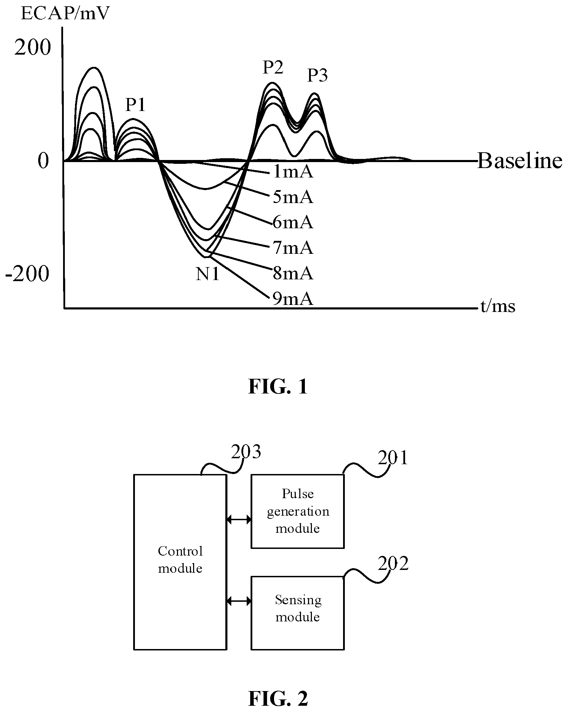

Fig. 1 is a schematic diagram of multiple ECAP curves under the pulse current stimulation with the same pulse frequency and different amplitudes. As can be seen from the figure, the ECAP curve generally has multiple peaks and troughs, such as first peak P1, first trough N1 after the first peak P1, second peak P2 after the first trough N1, and third peak P3 after the second peak P2. Obviously, the first peak P1, the second peak P2 and the third peak P3 are above the baseline, while the first trough N 1 is below the baseline. In addition, when the pulse current (i.e. the amplitude of the pulse) is greater than 5mA, the third peak P3 begins to appear, and the third peak P3 becomes larger and larger following the increase of the stimulation pulse. The inventor found that at the second peak P2, the proportion of the action potential caused by the stress of only the relatively thicker nerve fibers is larger, while at the third peak P3, the proportion of the action potential caused by the stress of the relatively finer nerve fibers is larger. Therefore, the convention peak-to-peak value of ECAP cannot fully reflect the real situation of nerve fibers of the body. A closed-loop feedback of the pulse generation module using the peak-to-peak value of ECAP as the parameter cannot effectively adjust the amplitude of the pulse, and cannot further control the action potential caused by the finer nerve fibers that may easily cause discomfort in patients to be within an appropriate range. In addition, the inventor also found that when the pulse current exceeds an amplitude threshold (such as SmA in the above figure), the peak-to-peak ratio of the evoked compound action potential between the third peak P3 and the second peak P2 has a linear relationship with the amplitude of the pulse.

Based on the above understanding, a first aspect of the embodiments of the present disclosure provides a nerve stimulation apparatus, which can be applied in a variety of medical environments using stimulation pulse, such as spinal cord electrical stimulation (SCS) systems, and dorsal root ganglion (DRG) electrical stimulation systems. The following is described by way of example with the present embodiment being applied in a spinal cord electrical stimulation scene.

As shown in Fig. 2, the first aspect of the embodiments of the present disclosure provides a nerve stimulation apparatus including: a pulse generation module 201, a sensing module 202 and a control module 203.

The pulse generation module 201 is configured to generate pulses according to an instruction from the control module 203.

The sensing module 202 is configured to sense an evoked compound action potential according to an instruction from the control module 203.

The control module 203 is configured to instruct the sensing module to sense the evoked compound action potential after a pulse is generated within a current pulse generation cycle, to obtain a peak-to-peak ratio of the evoked compound action potential, and to adjust, based on a determination that the peak-to-peak ratio of the evoked compound action potential is not within a comfort range, the amplitude of the pulse to be generated within a subsequent pulse generation cycle iteratively until the peak-to-peak ratio of the evoked compound action potential is within the comfort range; where the comfort range includes a dimension of amplitude of the pulse and a dimension of peak-to-peak ratio of the evoked compound action potential.

There are many options for the control module in practical applications, including: any one or combination of logic circuit, microcontroller unit (MCU), central processing unit (CPU), microprocessor, programmable logic control module (PLC), field programmable gate array (FPGA), programmable array logic (PAL), generic array logic (Gal) and complex programmable logic device (CPLD). The specific selection for the control module is not limited herein.

In this embodiment, the "comfort range" refers to the numerical range of the relevant parameters of the nerve stimulation apparatus and the corresponding body stress parameters when the pulse generated by the nerve stimulation apparatus places the patient in a comfortable state. For example, the relevant parameters of the nerve stimulation apparatus include the amplitude of the pulse, and the corresponding body stress parameters include the peak-to-peak value of ECAP. In this embodiment, the dimension of amplitude of the pulse within the comfort range includes the minimum amplitude of the pulse within the comfort range, and the maximum amplitude of the pulse within the comfort range. The dimension of peak-to-peak ratio of the evoked compound action potential within the comfort range includes the minimum peak-to-peak ratio of the evoked compound action potential within the comfort range corresponding to the pulse with the minimum amplitude within the comfort range, and the maximum peak-to-peak ratio of the evoked compound action potential within the comfort range corresponding to the pulse with the maximum amplitude within the comfort range. This embodiment has no special limitation on the method for "determining whether the peak-to-peak ratio of the evoked compound action potential in the current pulse generation cycle is within the comfort range". For example, the method for determining whether the peak-to-peak ratio of the evoked compound action potential in the current pulse generation cycle is within the comfort range may include: comparing the peak-to-peak ratio of the evoked compound action potential with the minimum peak-to-peak ratio of the evoked compound action potential within the comfort range and the maximum peak-to-peak ratio of the evoked compound action potential within the comfort range separately, and determining that the peak-to-peak ratio of the evoked compound action potential in the current pulse generation cycle is within the comfort range if the peak-to-peak ratio of the evoked compound action potential is greater than the minimum peak-to-peak ratio of the evoked compound action potential within the comfort range and less than the maximum peak-to-peak ratio of the evoked compound action potential within the comfort range. The dimension of amplitude of the pulse within the comfort range may further include one or more of all the amplitudes between the minimum amplitude of the pulse within the comfort range and the maximum amplitude of the pulse within the comfort range in addition to the minimum amplitude of the pulse within the comfort range and the maximum amplitude of the pulse within the comfort range. Correspondingly, the dimension of peak-to-peak ratio of the evoked compound action potential within the comfort range may further include one or more of the peak-to-peak ratios of ECAP corresponding to the pulses with the amplitude between the minimum amplitude of the pulse within the comfort range and the maximum amplitude of the pulse within the comfort range in addition to the minimum peak-to-peak ratio of the evoked compound action potential within the comfort range and the maximum peak-to-peak ratio of the evoked compound action potential within the comfort range. There may be one or more comfort ranges. For example, there may be a comfort range for each pulse frequency and/or body posture. The comfort range may also include other dimensions. For example, the comfort range may also include a dimension of the body posture, a dimension of the pulse frequency and other dimensions. That is, different pulse frequencies and/or different body postures, as well as the corresponding different comfort ranges are integrated into one-piece. This embodiment has no special restrictions on the specific manifestation of the comfort range. The comfort range may be represented and stored in any one of multidimensional array, curve, chart, queues, heap, stack, vector, linked list, tree and hash table that includes the above data. The comfort range may be generated by the doctor during the patient's visit and follow-up, or by the patient through a corresponding application. The comfort range may be stored in the control module or in a storage device communicatively connected with the control module.

In some embodiments, when the nerve stimulation apparatus for spinal cord electrical stimulation implanted into the patient starts working, the control module instructs the pulse generation module to periodically send, at a certain frequency, pulses with a specific amplitude to the patient according to the pre-stored or set parameters related to pulse generation and sensing of the evoked compound action potential. In addition, after the pulse generation module starts to generate pulses to the patient, the control module instructs the sensing module to start the sensing the evoked compound action potential to sense the reaction of the patient's nerve fibers to the pulses (i.e. evoked compound action potential, ECAP). Initial pulse may be set and generated according to the pulse parameters set by the doctor when the nerve stimulation apparatus is implanted or during the follow-up. The pulse generated by the nerve stimulation apparatus implanted in the patient will place the patient in a comfortable state. However, due to the influence of factors such as the patient's physical state or external environment, the existing pulse may no longer place the patient in a comfortable state. At this time, the control module needs to determine whether the patient is in a comfortable state according to the obtained peak-to-peak ratio of the evoked compound action potential, and adjust the pulse in time when the patient is not in a comfortable state.

For example, after the generation of the pulse within the current pulse generation cycle is finished, the control module instructs the sensing module to sense the ECAP caused by the pulse generated within the current pulse generation cycle, and obtains the ECAP information of the current pulse generation cycle through the sensing module, for example, obtains the potential value of the third peak P3 and the potential value of the second peak P2 in the ECAP curve corresponding to the current pulse generation cycle, and then obtains the peak-to-peak ratio of the evoked compound action potential of the current pulse generation cycle according to the ratio of the potential value of the third peak P3 to the potential value of the second peak P2. Then, the control module determines whether the peak-to-peak ratio of the evoked compound action potential of the current pulse generation cycle is within the comfort range. When the peak-to-peak ratio of the evoked compound action potential of the current pulse generation cycle is not within the comfort range, the control module adjusts the amplitude of the pulse to be generated within the subsequent pulse generation cycle iteratively until the peak-to-peak ratio of the evoked compound action potential is within the comfort range, so as to ensure that the pulse received by the patient can place the patient in a comfortable state and produce a good clinical therapeutic effect. The comfort range includes a dimension of amplitude of the pulse and a dimension of peak-to-peak ratio of the evoked compound action potential.

Further, the control module compares the peak-to-peak ratio of the evoked compound action potential with an upper limit and a lower limit of peak-to-peak ratio of the evoked compound action potential within the comfort range if the peak-to-peak ratio of the evoked compound action potential is not within the comfort range: the amplitude of the pulse to be generated within the subsequent pulse generation cycle is adjusted downward according to a preset step length if the peak-to-peak ratio of the evoked compound action potential is greater than the upper limit of the peak-to-peak ratio of the evoked compound action potential within the comfort range; and the amplitude of the pulse to be generated within the subsequent pulse generation cycle is adjusted upward according to the preset step length if the peak-to-peak ratio of the evoked compound action potential is less than the lower limit of the peak-to-peak ratio of the evoked compound action potential within the comfort range.

In an exemplary example, the control module obtains the peak-to-peak ratio of the evoked compound action potential of the current pulse generation cycle calculated from the potential value of the third peak P3 and the potential value of the second peak P2, and further adjusts the amplitude of the pulse to be generated within the subsequent pulse generation cycle according to the relationship between the peak-to-peak ratio of the evoked compound action potential of the current pulse generation cycle and the upper and lower limits of the peak-to-peak ratio of the evoked compound action potential within the comfort range when it is determined that the peak-to-peak ratio of the evoked compound action potential of the current pulse generation cycle is not within the comfort range according to the comfort range.

If the peak-to-peak ratio of the evoked compound action potential sensed in the current pulse generation cycle is greater than the upper limit of the peak-to-peak ratio of the evoked compound action potential within the comfort range, it is determined that the amplitude of the pulse generated within the current pulse generation cycle is too large, and it is required to decrease the amplitude of the subsequent pulse. The control module calculates the amplitude of the pulse to be generated within the subsequent pulse generation cycle according to the preset step length and the amplitude of the pulse generated within the current pulse generation cycle, so that the amplitude of the pulse to be generated within the subsequent pulse generation cycle decreases according to the preset step length. For example, the amplitude of the pulse generated within the current pulse generation cycle is denoted as A1 and the preset step length is denoted as A, and the amplitude of the pulse to be generated within the subsequent pulse generation cycle may be set to A2=A1-A, if the peak-to-peak ratio of the evoked compound action potential sensed in the current pulse generation cycle is greater than the upper limit of the peak-to-peak ratio of the evoked compound action potential within the comfort range.

If the peak-to-peak ratio of the evoked compound action potential sensed in the current pulse generation cycle is less than the lower limit of the peak-to-peak ratio of the evoked compound action potential within the comfort range, it is determined that the amplitude of the pulse generated within the current pulse generation cycle is too small, and it is required to increase the amplitude of the subsequent pulse. The control module calculates the amplitude of the pulse to be generated within the subsequent pulse generation cycle according to the preset step length and the amplitude of the pulse generated within the current pulse generation cycle, so that the amplitude of the pulse to be generated within the subsequent pulse generation cycle increases according to the preset step length. For example, the amplitude of the pulse generated within the current pulse generation cycle is denoted as A1 and the preset step length is denoted as A, and the amplitude of the pulse to be generated within the subsequent pulse generation cycle may be set to A2=A1+A, if the peak-to-peak ratio of the evoked compound action potential sensed in the current pulse generation cycle is less than the lower limit of the peak-to-peak ratio of the evoked compound action potential within the comfort range.

After adjusting the amplitude of the pulse to be generated within the subsequent pulse generation cycle, the control module instructs to generate the pulse with adjusted amplitude within the subsequent pulse generation cycle, and then instructs the sensing module to re-obtain a new evoked compound action potential caused by the pulse, and obtains the new peak-to-peak ratio of the evoked compound action potential therefrom. If the new peak-to-peak ratio of the evoked compound action potential is within the comfort range, the amplitude of the pulse will not be adjusted; if the new peak-to-peak ratio of the evoked compound action potential is still not within the comfort range, the amplitude of the pulse to be generated within the further subsequent pulse generation cycle is further adjusted iteratively until the peak-to-peak ratio of the evoked compound action potential is within the comfort range. Adjusting, according to the preset step length and the amplitude of the pulse generated within the current pulse generation cycle, the amplitude of the pulse to be generated within the subsequent pulse generation cycle makes the peak-to-peak ratio of the evoked compound action potential fall into the comfort range, so that the patient can be in a comfortable state and obtain a better therapeutic effect.

In an exemplary example, the preset step length is determined according to upper and lower limits of the amplitude of the pulse within the comfort range (which can be referred to as the upper and lower limits for short) and a preset scaling factor. The upper limit Amax and the lower limit Amin of the amplitude of the pulse are obtained according to the comfort range; and a preset scaling factor N is obtained; and then the preset step length for amplitude adjustment is calculated according to the upper limit Amax, the lower limit Amin and the scaling factor N.

For example, the amplitude change ΔA between the upper and lower limits of amplitude is calculated according to the upper limit Amax and the lower limit Amin, and then the step length, i.e. step=ΔA/N is obtained according to the preset scaling factor N. The control module sets the amplitude of the pulse within the subsequent pulse generation cycle Anext=Acur+step, or Anext=Acur-step, where Acur represents the amplitude of the pulse generated within the current pulse generation cycle. Here, on the premise that the upper and lower limits of the amplitude of the pulse are fixed, the scaling factor N determines the size of the step length step, and determines the number of cycles required to adjust the amplitude of the pulse into the comfort range and the fineness of the adjustment. The scaling factor N may be a value preset in the system or a value set by the doctor during the follow-up process. The step length of amplitude adjustment of the pulse is set according to the upper and lower limits of the amplitude of the pulse within the comfort range and the preset scaling factor, avoiding injuries to the patient due to the unreasonable amplitude adjustment setting in the process of amplitude adjustment of the pulse.

It should be noted that if the control module adjusts the amplitude of the pulse to be generated within the subsequent pulse generation cycle in the following way that the amplitude of the pulse is increased or decreased by one preset step length each time, multiple cycles of amplitude adjustment may be required to make the peak-to-peak ratio of the evoked compound action potential fall within the comfort range. Therefore, in an alternative embodiment, the control module may adjust the amplitude of the pulse to be generated within the subsequent pulse generation cycle in combination with the preset step length and an adjustment coefficient when the control module determines that the peak-to-peak ratio of the evoked compound action potential caused by the pulse generated within the current pulse generation cycle is not within the comfort range. For example, the control module sets the amplitude of the pulse to be generated within the subsequent pulse generation cycle to Anext=Acur+step×(N/2) if it is determined that the peak-to-peak ratio of the evoked compound action potential obtained in the current pulse generation cycle is less than the lower limit of the peak-to-peak ratio of the evoked compound action potential within the comfort range. After the pulse is generated within the subsequent pulse generation cycle, the control module again determines whether the peak-to-peak ratio of the evoked compound action potential obtained in the subsequent pulse generation cycle is within the comfort range. If the peak-to-peak ratio of the evoked compound action potential is still less than the lower limit of the peak-to-peak ratio of the evoked compound action potential within the comfort range, the amplitude of the pulse to be generated within the further subsequent pulse generation cycle is set to Anext'=Acur'+step, where Acur' is Anext. After the pulse is generated within the further subsequent pulse generation cycle, the control module again determines whether the obtained peak-to-peak ratio of the evoked compound action potential is within the comfort range. The above adjustment is iteratively performed until the peak-to-peak ratio of the evoked compound action potential falls into the comfort range. Similarly, the control module sets the amplitude of the pulse to be generated within the subsequent pulse generation cycle to Anext=Acur-step×(N/2) if it is determined that the peak-to-peak ratio of the evoked compound action potential obtained in the current pulse generation cycle is less than the upper limit of the peak-to-peak ratio of the evoked compound action potential within the comfort range. After the pulse is generated within the subsequent pulse generation cycle, the control module again determines whether the peak-to-peak ratio of the evoked compound action potential obtained in the subsequent pulse generation cycle is within the comfort range. If the peak-to-peak ratio of the evoked compound action potential is still greater than the upper limit of the peak-to-peak ratio of the evoked compound action potential within the comfort range, the amplitude of the pulse to be generated within the further subsequent pulse generation cycle is set to Anext'=Acur'-step, where Acur' is Anext. After the pulse is generated within the further subsequent pulse generation cycle, the control module again determines whether the obtained peak-to-peak ratio of the evoked compound action potential is within the comfort range. The above adjustment is iteratively performed until the peak-to-peak ratio of the evoked compound action potential falls into the comfort range. In this embodiment, the control module can rapidly adjust the amplitude of the pulse, rapidly relieve the discomfort of the patient, and finely adjust the amplitude of the pulse at the same time, so as to achieve a better therapeutic effect.

In a more radical alternative, after the pulse is generated within the subsequent pulse generation cycle, the amplitude of the pulse to be generated within the further subsequent pulse generation cycle is set to Anext'=Acur'+step × (N/4) if the control module determines that the peak-to-peak ratio of the evoked compound action potential obtained in the subsequent pulse generation cycle is still less than the lower limit of the peak-to-peak ratio of the evoked compound action potential within the comfort range. After the pulse is generated within the further subsequent pulse generation cycle, the control module again determines whether the peak-to-peak ratio of the evoked compound action potential obtained in the further subsequent pulse generation cycle is within the comfort range. Similarly, the amplitude of the pulse to be generated within the further subsequent pulse generation cycle is set to Anext'=Acur'-step × (N/4) if the control module determines that the peak-to-peak ratio of the evoked compound action potential obtained in the subsequent pulse generation cycle is still greater than the upper limit of the peak-to-peak ratio of the evoked compound action potential within the comfort range. After the pulse is generated within the further subsequent pulse generation cycle, the control module again determines whether the peak-to-peak ratio of the evoked compound action potential obtained in the further subsequent pulse generation cycle is within the comfort range. In this embodiment, the control module can more quickly adjust the amplitude of the pulse and quickly relieve the discomfort of the patient.

It should be noted that according to the present disclosure, no special limitation is imposed on the way to obtain the potential value of the second peak P2 and the potential value of the third peak P3. In an exemplary example, the sensing module samples the potential value of the evoked compound action potential at a certain sampling frequency. The control module first stores each potential value and associated information including index information in a certain form, and then obtains the potential value of the second peak P2 and the potential value of the third peak P3 according to all the potential values obtained from the sampling and the corresponding associated information, and determines the peak-to-peak ratio of the evoked compound action potential according to the potential value of the third peak P3 and the potential value of the second peak P2. The "index information" is used to indicate the sequence of the potential value relative to other potential values in the sampling process. In this exemplary example, the numerical value of all potential values in the ECAP data set and the number of data (potential values) in the data set are clear when obtaining the second peak P2 and the third peak P3. Therefore, the potential values of the third peak P3 and the second peak P2 can be obtained accurately to ensure the accuracy of the obtained peak-to-peak ratio of the evoked compound action potential.

As shown in Fig. 1, according to the characteristics of the ECAP curve, the trough with the largest absolute value below the baseline is the first trough N1. Accordingly, the potential value of the first trough N1 is the minimum potential value in the ECAP curve. Generally, the first trough in the ECAP curve is the first trough N1. The second peak P2 is the largest peak after the first trough N1. Accordingly, the potential value of the second peak P2 is the maximum potential value in the ECAP curve after the first trough N1. The third peak P3 is the largest peak after the second peak P2. Accordingly, the potential value of the third peak P3 is the maximum potential value in the ECAP curve after the immediate trough after the second peak P2.

In an exemplary example, each potential value and the corresponding associated information form ECAP sampling data, and all the ECAP sampling data form an ECAP data set, in which the associated information includes index information for indicating the sequence in which each potential value is sampled and stored. The control module first traverses all ECAP sampling data in the ECAP data set, takes the potential value of the ECAP sampling data with the smallest potential value in the ECAP data set as the potential value of the first trough N1, and takes the index of the ECAP sampling data with the smallest potential value in the ECAP data set as the first index; then takes all ECAP sampling data with the index after the first index as a first subset, traverses all ECAP sampling data in the first subset, takes the potential value of the ECAP sampling data with the largest potential value in the first subset as the potential value of the second peak P2, and takes the index of the ECAP sampling data with the largest potential value in the first subset as a second index; then determines, according to the second index, a third index, and takes the potential value of the ECAP sampling data with the largest potential value, among all ECAP sampling data with the index after the third index, as the potential value of the third peak P3. The control module takes all ECAP sampling data with the index after the third index as a second subset, traverses all ECAP sampling data in the second subset, and takes the potential value of the ECAP sampling data with the largest potential value in the second subset as the potential value of the third peak P3.

For example, the control module traverses all ECAP sampling data in the ECAP data set and compares the potential value of each ECAP sampling data in the ECAP data set with a first temporary number: if the potential value of the current ECAP sampling data is less, updates the value of the first temporary number to the current potential value, and records the index of the current ECAP sampling data, then compares the potential value of the next ECAP sampling data with the first temporary number. The above process is performed iteratively until all the ECAP sampling data in the ECAP data set has been traversed. After the above process, the first temporary number is taken as the potential value of the first trough N1, and the corresponding index as the first index. Then, the control module takes all ECAP sampling data with the index after the first index as the first subset, traverses all ECAP sampling data in the first subset, and compares the potential value of each ECAP sampling data in the first subset with a second temporary number: if the potential value of the current ECAP sampling data is greater, updates the value of the second temporary number to the current potential value, and records the index of the current ECAP sampling data, then compares the potential value of the next ECAP sampling data with the second temporary number. The above process is performed iteratively until all the ECAP sampling data in the first subset has been traversed. After the above process, the second temporary number is taken as the potential value of the second peak P2, and the corresponding index as the second index. After obtaining the data of the second peak P2, the control module determines the third index according to the characteristics of the ECAP curve and the second index. The third index is such configured that among all ECAP sampling data with the index after the third index, the potential value of the third peak P3 is the potential value of the ECAP sampling data with the largest potential value. Further, all ECAP sampling data with index after the third index are taken as the second subset. A third temporary number is taken as the potential value of the third peak P3 in a similar way to that for determining the data of the second peak P2. The third peak P3 and the second peak P2 are accurately determined by traversing data in a specific range and searching for the extreme value, so that the peak-to-peak ratio of the evoked compound action potential is accurately determined, which facilitates accurate judgment of the current state of the patient.

It is worth mentioning that this embodiment does not limit the form of index in the ECAP sampling data, as long as it can characterize the sequence in which each ECAP sampling data is sampled. The index in each ECAP sampling data may be in the form of absolute value. For example, the index is represented by data sampling time. Alternatively, the index may be in the form of relative value. For example, the index is represented by a natural number, the index of the first sampled data is 1, the index of the second data is 2, and so on. In this embodiment, there is no special limitation on the form of storage of ECAP sampling data. For example, the ECAP sampling data may be stored in the form of queue, binary tree, array, graph and table.

As shown in Fig. 1, generally speaking, in the ECAP curve, the third peak P3 is smaller than the second peak P2. Therefore, the maximum value of all potential values after the second index cannot be simply taken as the potential value of the third peak P3, otherwise it is likely that the potential value of the ECAP sampling data near the second peak P2 will be wrongly taken as the potential value of the third peak P3. Therefore, it is necessary to combine the characteristics of the ECAP curve to obtain the potential value of the third peak P3.

In an exemplary example, the control module combines the obtained second index with a preset index offset to obtain the third index. The index offset here may be preset when the system pre-install, or set by the doctor during implantation or follow-up based on the obtained ECAP curve or based on clinical experience, and this embodiment does not impose any limitation on this.

In an alternative example, in view of the characteristics of the ECAP curve, the ECAP sampling data corresponding to the immediate trough after the second peak P2 is obtained, and the index in this ECAP sampling data is taken as the third index. In this example, the control module takes all ECAP sampling data with the index after the second index as the third subset, traverses all ECAP sampling data in the third subset according to the index sequence, and subtracts the potential value of the previous ECAP sampling data from the potential value of each ECAP sampling data in the third subset to obtain a difference. When the difference changes from negative to positive, the potential value of the current ECAP sampling data is taken as the potential value of the immediate trough after the second peak P2, and the index of the current ECAP sampling data is taken as the third index. Further, in order to avoid noise, when the difference changes from negative to positive, the control module continues to traverse the third subset, and accumulates the number of positive differences. If the accumulated value exceeds a preset threshold, the potential value of the ECAP sampling data at which the difference changes from negative to positive is taken as the potential value of the immediate trough after the second peak P2, and the index of this ECAP sampling data is taken as the third index.

In another embodiments, when the sensing module obtains the potential value of the evoked compound action potential at a certain sampling frequency, the control module obtains the potential values of the third peak P3 and the second peak P2 according to the real-time sampled potential values of the evoked compound action potential. In this embodiment, to avoid the need to store a large number of ECAP sampling data, the control module can directly obtain the potential values of the third peak P3 and the second peak P2 according to the real-time sampled potential values of the evoked compound action potential, so as to reduce the storage burden.

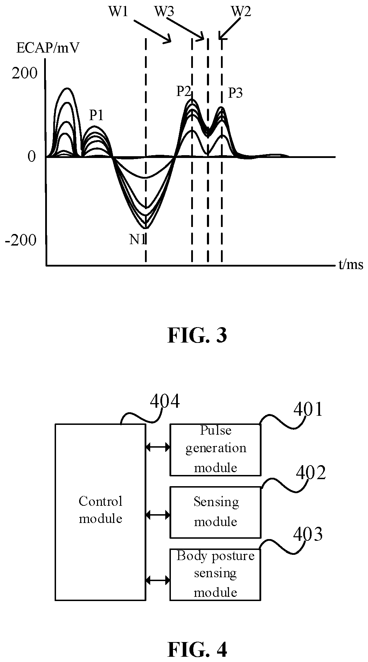

Fig. 3 is the characteristic diagram of ECAP curves caused by stimulation pulses. As shown in Fig. 3, according to the characteristics of the curve, it can be seen that from the first trough N1 to the second peak P2 (i.e. W1 region), the potential value of the evoked compound action potential is approximately incremented with time; and from the immediate trough after the second peak P2 to the third peak P3 (i.e. W2 region), the potential value of the evoked compound action potential is approximately incremented with time. Further, from the second peak P2 to the immediate trough after the second peak P2 (i.e. W3 region), the potential value of the evoked compound action potential is approximately decremented with time. Based on the above knowledge, the potential value of the second peak P2 and the potential value of the third peak P3 can be obtained by obtaining the maximum value of the W1 region and the maximum value of the W2 region. Therefore, when the control module determines the potential values of the second peak P2 and the third peak P3 according to the real-time sampled potential values, it can first determine the approximate starting positions of W1 and W2 regions, and further determine the potential values of the second peak P2 and the third peak P3 by using the law of the potential value changes in the region. That is, the control module obtains the minimum value as the potential value of the first trough N1 according to the real-time sampled potential values, obtains the first maximum value as the potential value of the second peak P2 according to the real-time sampled potential values after obtaining the potential value of the first trough N1, and obtains the second maximum value as the potential value of the third peak P3 according to the real-time sampled potential values after obtaining the immediate trough after the second peak P2. Here, "the first maximum value" and "the second maximum value" are only used to distinguish the maximum values of the two regions, rather than emphasizing which is the greater and which is the smaller.

Specifically, the nerve stimulation apparatus further includes a first register, a second register, a third register and a counter. The first register, the second register, the third register and the counter may be used as independent modules or as a part of the control module, which is not specifically limited herein.

After obtaining the real-time sampled potential value of the evoked compound action potential, the control module compares the real-time sampled potential value with a first potential value in the first register: when the real-time sampled potential value is greater than the first potential value, the counting of the counter accumulates; and when the real-time sampled potential value is less than the first potential value, the real-time sampled potential value is stored in the first register as the new first potential value, and the counting of the counter is cleared. Then, the control module compares the newly sampled potential value with the first potential value in the first register until the counting of the counter reaches a first preset threshold. When the counting of the counter reaches the first preset threshold, the control module takes the potential value currently stored in the first register as the potential value of the first trough N1. Since the first trough N1 is the minimum value in the whole ECAP curve, it can be easily obtained through this method. At the same time, the counter accumulation method is used to confirm that the value in the register is the extreme value, avoiding the interference caused by noisy peaks.

After determining the potential value of the first trough N1, the control module continues to determine the potential value of the second peak P2. The control module compares the real-time sampled potential value of the evoked compound action potential with a second potential value pre-stored in the second register: when the real-time sampled potential value is less than the second potential value, the counting of the counter accumulates; and when the real-time sampled potential value is greater than the second potential value, the real-time sampled potential value is stored in the second register as the new second potential value, and the counting of the counter is cleared. Then, the control module compares the newly sampled potential value with the second potential value in the second register until the counting of the counter reaches a second preset threshold. When the counting of the counter reaches the second preset threshold, the control module takes the potential value currently stored in the second register as the potential value of the second peak P2.

After determining the potential value of the second peak P2, the control module continues to determine the potential value of the third peak P3 from the immediate trough after the second peak P2. The control module compares the real-time sampled potential value with a third potential value pre-stored in the third register: when the real-time sampled potential value is less than the third potential value, the counting of the counter accumulates; and when the real-time sampled potential value is greater than the third potential value, the real-time sampled potential value is stored in the third register as the new third potential value, and the counting of the counter is cleared. Then, the control module compares the newly sampled potential value with the third potential value in the third register until the counting of the counter reaches a third preset threshold. When the counting of the counter reaches the third preset threshold, the control module takes the potential value currently stored in the third register as the potential value of the third peak P3. In this embodiment, "from the immediate trough after the second peak P2" should not be narrowly understood as from the point where the trough is located, but should be understood as from the potential value lower than the potential value of the third peak P3 sampled after the second peak P2 according to the characteristics of the ECAP curve, where the potential value of the third peak P3 herein may be provided based on statistics or operator experience.

According to the characteristics of the ECAP curve, the method of comparing the real-time sampled evoked compound action potential with the potential value stored in each register according to the sampling sequence, and then determining the potential values of the second peak P2 and the third peak P3 can ensure the accuracy of the peak-to-peak ratio of the evoked compound action potential while avoiding the storage of a large number of evoked compound action potential data.

In the above embodiments, there may be one or more counters. If there is only one counter, it is required to clear the counter before determining the potential value of the trough or peak. Similarly, the register is required to be assigned a preset value before determining the potential value of the trough or peak. The preset value here may be a specific value, such as zero, or the potential value obtained in the first sampling.

In the above embodiments, there may be only one register, that is, the register is used to store the first potential value when obtaining the first trough N1, to store the second potential value when obtaining the second peak P2, and to store the third potential value when obtaining the third peak P3.

This embodiment has no special limitation on the method of obtaining the potential value of the immediate trough after the control module obtains the potential value of the second peak P2.

In an exemplary example, after obtaining the potential value of the second peak P2, the control module is further configured to take the real-time sampled potential value as the potential value of the immediate trough after the second peak P2 when a real-time offset with the second peak P2 meets a preset offset threshold, and determine the potential value of the third peak P3 from then on. After obtaining the potential value of the second peak P2, the control module reads out the preset offset threshold obtained or stored in advance, determines whether the real-time offset between the real-time evoked compound action potential and the second peak P2 meets the preset offset threshold, and takes the potential value obtained at this time as the potential value of the immediate trough after the second peak P2 when the real-time offset meets the preset offset threshold, and determine the potential value of the third peak P3 from then on. The way of obtaining the trough immediately after the second peak P2 after the offset of the preset offset threshold makes it relatively easy to know the trough immediately after the second peak P2, which requires a small number of operations, reduces the system overhead and saves power. In this embodiment, the specific form of the offset may be time. That is, the control module takes the potential value sampled when a preset time has elapsed after the potential value of the second peak P2 is obtained as the potential value of the trough immediately after the second peak P2. The specific form of the offset may be data volume. That is, after the potential value of the second peak P2 is obtained, the control module controls the sensing module to continue sampling potential values, and after the mount of the sampled potential values reaches the preset data volume, a potential value in the subsequent sampling is taken as the potential value of the trough immediately after the second peak P2. The offset may also have other forms, which is not limited in this embodiment.

It is worth mentioning that the preset offset threshold may be preset in factory, or set by the doctor during implantation or follow-up based on the obtained ECAP curve or based on clinical experience, and this embodiment does not impose any limitation on this.

In an alternative example, after obtaining the potential value of the second peak P2, the control module may further obtain a minimum value according to the real-time sampled potential valves and take the minimum valve as the potential value of the trough immediately after the second peak P2.

The control module may further include a fourth register. After determining the potential value of the second peak P2, the control module compares the real-time sampled potential value of the evoked compound action potential with a fourth potential value in the fourth register: when the real-time sampled potential value is greater than the fourth potential value, the counting of the counter accumulates; and when the real-time sampled potential value is not greater than the fourth potential value, the real-time sampled potential value is stored in the fourth register as the new fourth potential value, and the counting of the counter is cleared. Then, the control module compares the newly sampled potential value with the fourth potential value in the fourth register until the counting of the counter reaches a fourth preset threshold. When the counting of the counter reaches the fourth preset threshold, the control module takes the potential value currently stored in the fourth register as the potential value of the immediate trough after the second peak P2.

The potential value of the immediate trough after the second peak P2 can be accurately obtained by comparing the real-time sampled potential value of the evoked compound action potential with the fourth potential value, which lays the foundation for obtaining the accurate potential value of the third peak P3.

Furthermore, the control module is further configured to obtain different comfort ranges according to different body postures and/or different pulse frequencies. The comfort range of the patient varies if the patient is in different postures or subjected to stimulation pulses with different frequencies. The control module obtains the corresponding comfort range according to the frequency of the pulse generated within the current pulse generation cycle and the patient's body posture in the current pulse generation cycle, and then determines whether the peak-to-peak ratio of the evoked compound action potential is within the corresponding comfort range. For example, after obtaining the ECAP information of a pulse generation cycle, the control module obtains, according to the frequency of the pulse generated within the current pulse generation cycle and/or the patient's body posture in the current pulse generation cycle, a corresponding comfort range for subsequent comparison with the obtained peak-to-peak ratio of the evoked compound action potential. The patient's feelings can be accurately judged by providing different comfort ranges according to pulse frequency and/or body posture, which ensures the accuracy of adjustment of stimulation pulse. Obviously, the control module may obtain the comfort range before generating pulses or obtaining the ECAP information. This embodiment has no special limitation on the sequence. While the control module adjusts the amplitude of the pulse to make the peak-to-peak ratio of the evoked compound action potential fall into the comfort range, if the comfort range changes due to the change of pulse frequency or body posture, the control module stops the current adjustment process, re-obtains the new peak-to-peak ratio of the evoked compound action potential, and determines whether the new peak-to-peak ratio of the evoked compound action potential is within the new comfort range. If it is not in the new comfort range, the control module starts a new adjustment. In an alternative embodiment, as previously described, the comfort range may also include dimensions such as body posture and pulse frequency. Different pulse frequencies and/or different body postures, as well as the corresponding different comfort ranges are integrated into one-piece. In this case, the control module obtains, according to different body postures and/or different pulse frequencies, the upper and lower limits of the amplitude of the pulse and the upper and lower limits of the peak-to-peak ratio of the evoked compound action potential corresponding to the body posture and/or the frequency of the pulse within the comfort range.

The nerve stimulation apparatus further includes: a body posture sensing module; where the body posture sensing module is communicatively connected with the control module to sense the current body posture; and the control module is further configured to obtain the body posture through the body posture sensing module, so as to provide the comfort range corresponding to the body posture. Referring to Fig. 4, the nerve stimulation apparatus includes a pulse generation module 401, a sensing module 402, a body posture sensing module403 and a control module 404, where the control module 404 is communicatively connected with the others. When the nerve stimulation apparatus enters the working state, the control module obtains the current body posture of the patient according to the body posture data sensed by the body posture sensing module, and calls, according to the obtained body posture or according to the body posture and the frequency of the pulse generated within the current pulse generation cycle, the corresponding comfort range before determining whether the sensed peak-to-peak ratio of the evoked compound action potential is within the comfort range, and then compares the obtained peak-to-peak ratio of the evoked compound action potential with the comfort range, and determines whether the amplitude of the pulse to be generated within the subsequent pulse generation cycle needs to be adjusted according to the comparison result. The body posture sensing module is provided for sensing the body posture. The corresponding specific comfort range can be accurately obtained according to the sensed body posture, so as to accurately judge whether the patient is in a comfortable state and ensure the accuracy of adjustment of pulse. In an alternative embodiment, the control module obtains the body posture through the body posture sensing module to obtain the upper and lower limits of the amplitude of the pulse and the upper and lower limits of the peak-to-peak ratio of the evoked compound action potential corresponding to the body posture within the comfort range.

It should be noted that according to the present disclosure, no special limitation is imposed on a pulse generation mode of the nerve stimulation apparatus. In some embodiments, the pulse generation mode of the nerve stimulation apparatus includes tonic spiking mode, and tonic bursting mode. In a case that the pulse generation mode of the nerve stimulation apparatus in this embodiment is the tonic bursting mode, the control module instructs the sensing module to sense the evoked compound action potential when it instructs the pulse generation module to generate the last pulse in a cluster of pulses, to obtain the peak-to-peak ratio of the evoked compound action potential, and determines whether the obtained peak-to-peak ratio of the evoked compound action potential is within the comfort range. For example, the control module instructs the pulse generation module to generate a cluster of pulses at a certain frequency, such as 50Hz. The frequency of the pulses in the cluster is, for example, 400Hz to 600Hz. When the control module instructs the pulse generation module to generate the last pulse in a cluster of pulses, the control module instructs the sensing module to sense the evoked compound action potential to obtain the peak-to-peak ratio of the evoked compound action potential, and determines whether the peak-to-peak ratio of the evoked compound action potential is within the comfort range. In a case that the pulse generation mode of the nerve stimulation apparatus in this embodiment is the tonic spiking mode, the control module instructs the sensing module to sense the evoked compound action potential when it instructs the pulse generation module to generate the pulse, to obtain the peak-to-peak ratio of the evoked compound action potential, and determines whether the obtained peak-to-peak ratio of the evoked compound action potential is within the comfort range. For example, the control module instructs the pulse generation module to generate pulses at a frequency below 150Hz, for example, at a frequency of 50Hz. After the control module instructs the generation of the pulses, it controls the sensing module to sense the evoked compound action potential to obtain the peak-to-peak ratio of the evoked compound action potential.

In some embodiments, the control module further obtains the peak-to-peak value of ECAP while obtaining the peak-to-peak ratio of the evoked compound action potential. When the peak-to-peak ratio of the evoked compound action potential and the peak-to-peak value of ECAP are not within the comfort range, the control module adjusts the amplitude of the pulse to be generated within the subsequent pulse generation cycle iteratively until the peak-to-peak ratio of the evoked compound action potential and the peak-to-peak value of ECAP are within the comfort range; where the comfort range further includes a dimension of peak-to-peak value of ECAP in addition to the dimension of amplitude of the pulse and the dimension of peak-to-peak ratio of the evoked compound action potential.

It should be noted that the "peak-to-peak value of ECAP" here refers to the difference between the potential value of the second peak P2 and the potential value of the first trough N1. In this embodiment, the dimension of peak-to-peak value of ECAP within the comfort range includes the minimum peak-to-peak value of ECAP within the comfort range corresponding to the pulse with the minimum amplitude within the comfort range, and the maximum peak-to-peak value of ECAP within the comfort range corresponding to the pulse with the maximum amplitude within the comfort range. This embodiment has no special limitation on the method for "determining whether the peak-to-peak value of ECAP in the current pulse generation cycle is within the comfort range". For example, the method for determining whether the peak-to-peak value of ECAP in the current pulse generation cycle is within the comfort range may include: comparing the peak-to-peak value of ECAP with the minimum peak-to-peak value of ECAP within the comfort range and the maximum peak-to-peak value of ECAP within the comfort range separately, and determining that the peak-to-peak value of ECAP in the current pulse generation cycle is within the comfort range if the peak-to-peak value of ECAP is greater than the minimum peak-to-peak value of ECAP within the comfort range and less than the maximum peak-to-peak value of ECAP within the comfort range.

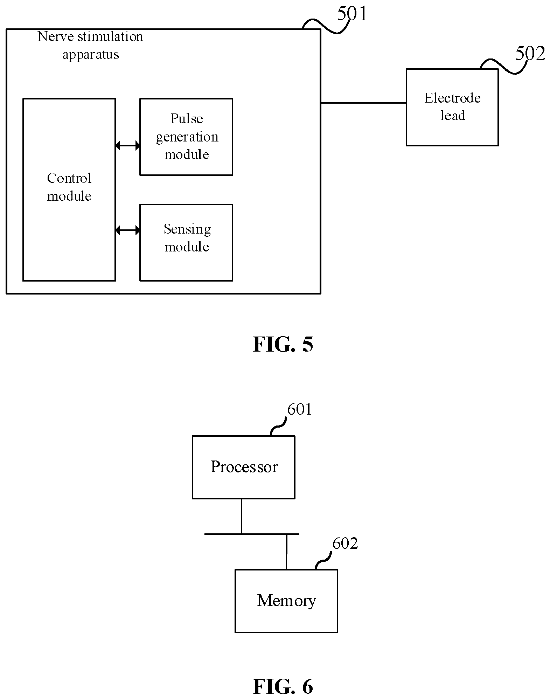

As shown in Fig. 5, another aspect of the embodiments of the present disclosure provides a nerve stimulation system including the nerve stimulation apparatus 501 as described above and an electrode lead 502 electrically connected with the nerve stimulation apparatus. The electrode lead includes multiple stimulation electrodes and sensing electrodes, where the stimulation electrodes are configured to transmit the stimulation pulse into the patient, and the sensing electrodes are configured to transmit the evoked compound action potential caused by the stimulation pulse to the sensing module. The stimulation electrodes may be ring electrodes or segmented electrodes. This embodiment has no special limitation on the type of electrode. For example, the electrode lead is an electrode lead provided with ring electrodes at distal end. For another example, the electrode lead is an electrode lead provided with segmented electrodes at a distal end. For yet another example, the electrode lead is an electrode lead provided with ring electrodes and segmented electrodes at a distal end.

Another aspect of the embodiments of the present disclosure provides a method applied at a nerve stimulation apparatus including:

instructing to sense an evoked compound action potential after the pulse generation module generates a pulse within a current pulse generation cycle;

obtaining a peak-to-peak ratio of the evoked compound action potential, and adjustment, based on a determination that the peak-to-peak ratio of the evoked compound action potential is not within a comfort range, the amplitude of the pulse to be generated by the pulse generation module within a subsequent pulse generation cycle iteratively until the peak-to-peak ratio of the evoked compound action potential is within the comfort range; where the comfort range includes a dimension of amplitude of the pulse and a dimension of peak-to-peak ratio of the evoked compound action potential.

It is not difficult to find that this embodiment is a method embodiment corresponding to the device embodiment, and this embodiment may be implemented in cooperation with the device embodiment. The related technical details mentioned in the device embodiment are still effective in this embodiment, and are not repeated here in order to reduce repetition. Accordingly, the related technical details mentioned in this embodiment may also be applied to the device embodiment.

As shown in Fig. 6, another aspect of the embodiments of the present disclosure further provides an electronic device including: at least one processor 601; and a memory 602 communicatively connected to the at least one processor 601; where the memory 602 stores instructions executable by the at least one processor 601, and the instructions, when executed by the at least one processor 601, cause the at least one processor 601 to implement a method applied at a nerve stimulation apparatus. The control method includes: instructing to sense an evoked compound action potential after the pulse generation module generates a pulse within a current pulse generation cycle; obtaining a peak-to-peak ratio of the evoked compound action potential, and adjustment, based on a determination that the peak-to-peak ratio of the evoked compound action potential is not within a comfort range, the amplitude of the pulse to be generated by the pulse generation module within a subsequent pulse generation cycle iteratively until the peak-to-peak ratio of the evoked compound action potential is within the comfort range; where the comfort range includes a dimension of amplitude of the pulse and a dimension of peak-to-peak ratio of the evoked compound action potential.

The memory 602 and the at least one processor 601 are connected using a bus, the bus may include any number of interconnected data lines and bridges, and the bus connects various circuits of the at least one processor 601 and the memory 602 together. The bus may also connect together various other circuits, such as a peripheral device, a voltage regulator, and a power management circuit, etc., which are well known in the art, and therefore are not further described herein. A bus interface provides an interface between the bus and a transceiver. The transceiver may be an element, or may be a plurality of elements, such as a plurality of receivers and transmitters, providing units for communicating with various other devices on a transmission medium. The data processed by the at least one processor 601 is transmitted on a wireless medium through an antenna, and further, the antenna receives the data and transmits the data to the at least one processor 601.

The at least one processor 601 is responsible for managing buses and general processing, and may also provide various functions including timing, peripheral interface, voltage adjustment, power management, and other control functions. The memory 602 may be configured to store data used by the at least one processor 601 when performing operations.

The embodiments of the present disclosure further provide a computer-readable storage medium storing a computer program. The computer program, when executed by a processor, causes the processor to implement a method applied at a nerve stimulation apparatus. The control method includes: instructing to sense an evoked compound action potential after the pulse generation module generates a pulse within a current pulse generation cycle; obtaining a peak-to-peak ratio of the evoked compound action potential, and adjusting, based on a determination that the peak-to-peak ratio of the evoked compound action potential is not within a comfort range, the amplitude of the pulse to be generated by the pulse generation module within a subsequent pulse generation cycle iteratively until the peak-to-peak ratio of the evoked compound action potential is within the comfort range; where the comfort range includes a dimension of amplitude of the pulse and a dimension of peak-to-peak ratio of the evoked compound action potential.

That is, a person skilled in the art may understand that all or some of operations in the method in the above embodiments may be completed by a program instructing related hardware, and the program may be stored in a storage medium and includes several instructions for enabling a device (which may be a single-chip microcomputer, a chip, etc.) or a processor to perform all or some of the operations in the method described in the embodiments of the present disclosure. The above storage medium includes various media that can store program codes, such as a USB flash disk, a mobile hard disk, a read-only memory (ROM), a random access memory (RAM), a magnetic disk, or an optical disc, etc.

It should be understood by those of ordinary skill in the art that the above embodiments are specific embodiments for implementing the present disclosure, and in practical applications, various changes may be made in form and detail thereto without departing from the spirit and scope of the present disclosure.

## Claims

1. A nerve stimulation apparatus, comprising: a pulse generation module, a sensing module and a control module;
wherein the pulse generation module is configured to generate pulses according to an instruction from the control module;
the sensing module is configured to sense an evoked compound action potential according to an instruction from the control module; and
the control module is configured to instruct the sensing module to sense the evoked compound action potential after a pulse is generated within a current pulse generation cycle, to obtain a peak-to-peak ratio of the evoked compound action potential, and to adjust, based on a determination that the peak-to-peak ratio of the evoked compound action potential is not within a comfort range, the amplitude of the pulse to be generated within a subsequent pulse generation cycle iteratively until the peak-to-peak ratio of the evoked compound action potential is within the comfort range; wherein the comfort range includes a dimension of amplitude of the pulse and a dimension of peak-to-peak ratio of the evoked compound action potential.

2. The nerve stimulation apparatus according to claim 1, wherein the control module is configured to compare the peak-to-peak ratio of the evoked compound action potential with an upper limit and a lower limit of peak-to-peak ratio of the evoked compound action potential within the comfort range based on a determination that the peak-to-peak ratio of the evoked compound action potential is not within the comfort range:
the amplitude of the pulse to be generated within the subsequent pulse generation cycle is adjusted downward according to a preset step length based on a determination that the peak-to-peak ratio of the evoked compound action potential is greater than the upper limit of peak-to-peak ratio of the evoked compound action potential within the comfort range; and
the amplitude of the pulse to be generated within the subsequent pulse generation cycle is adjusted upward according to the preset step length based on a determination that the peak-to-peak ratio of the evoked compound action potential is less than the lower limit of peak-to-peak ratio of the evoked compound action potential within the comfort range.

3. The nerve stimulation apparatus according to claim 2, wherein the preset step length is determined according to upper and lower limits of the amplitude of the pulse within the comfort range and a preset scaling factor.

4. The nerve stimulation apparatus according to claim 2 or 3, wherein the amplitude of the pulse to be generated within the subsequent pulse generation cycle is adjusted downward according to the preset step length, including: the amplitude Anext of the pulse to be generated within the subsequent pulse generation cycle is adjusted in accordance with the following formula: Anext=Acur-step, wherein Acur represents the amplitude of the pulse generated in the current pulse generation cycle, and step represents the preset step length;
or, the amplitude Anext of the pulse to be generated within the subsequent pulse generation cycle is adjusted in accordance with the following formula: Anext=Acur-step×(N/2), wherein Acur represents the amplitude of the pulse generated in the current pulse generation cycle, step represents the preset step length, and N represents the preset scaling factor;
or, the amplitude Anext of the pulse to be generated within the subsequent pulse generation cycle is adjusted in accordance with the following formula: Anext=Acur-step×(N/4), wherein Acur represents the amplitude of the pulse generated in the current pulse generation cycle, step represents the preset step length, and N represents the preset scaling factor.

5. The nerve stimulation apparatus according to claim 2 or 3, wherein the amplitude of the pulse to be generated within the subsequent pulse generation cycle is adjusted upward according to the preset step length, including: the amplitude Anext of the pulse to be generated within the subsequent pulse generation cycle is adjusted in accordance with the following formula: Anext=Acur+step, wherein Acur represents the amplitude of the pulse generated in the current pulse generation cycle, and step represents the preset step length;
or, the amplitude Anext of the pulse to be generated within the subsequent pulse generation cycle is adjusted in accordance with the following formula: Anext=Acur+step×(N/2), wherein Acur represents the amplitude of the pulse generated in the current pulse generation cycle, step represents the preset step length, and N represents the preset scaling factor;
or, the amplitude Anext of the pulse to be generated within the subsequent pulse generation cycle is adjusted in accordance with the following formula: Anext=Acur+step×(N/4), wherein Acur represents the amplitude of the pulse generated in the current pulse generation cycle, step represents the preset step length, and N represents the preset scaling factor.

6. The nerve stimulation apparatus according to any one of claims 1 to 3, wherein the control module is further configured to obtain different comfort ranges according to different body postures and/or different pulse frequencies, or to obtain, according to different body postures and/or different pulse frequencies, the upper and lower limits of the amplitude of the pulse and the upper and lower limits of the peak-to-peak ratio of the evoked compound action potential corresponding to the body posture and/or the frequency of the pulse within the comfort range.

7. The nerve stimulation apparatus according to any one of claims 1 to 6, further comprising: a body posture sensing module;
wherein the body posture sensing module is communicatively connected with the control module to sense a current body posture; and
the control module is further configured to obtain the body posture through the body posture sensing module to obtain the comfort range corresponding to the body posture, or to obtain the body posture through the body posture sensing module to obtain the upper and lower limits of the amplitude of the pulse and the upper and lower limits of the peak-to-peak ratio of the evoked compound action potential corresponding to the body posture within the comfort range.

8. The nerve stimulation apparatus according to any one of claims 1 to 7, wherein the control module is further configured to:
store the potential values and associated information of the evoked compound action potential obtained by sampling after instructing the sensing module to sense the evoked compound action potential, wherein the associated information includes index information for indicating a sampling sequence; and
obtain a potential value of a second peak P2 and a potential value of a third peak P3 according to the potential values and the associated information of the evoked compound action potential.

9. The nerve stimulation apparatus according to claim 8, wherein the control module is further configured to first traverse all evoked compound action potential (ECAP) sampling data, each of which formed by the potential value of the evoked compound action potential and the corresponding associated information in the ECAP data set formed by all the ECAP sampling data, take the potential value of the ECAP sampling data with the smallest potential value in the ECAP data set as the potential value of a first trough, and take the index of the ECAP sampling data with the smallest potential value in the ECAP data set as a first index; then take all ECAP sampling data with the index after the first index as a first subset, traverse all ECAP sampling data in the first subset, take the potential value of the ECAP sampling data with the largest potential value in the first subset as the potential value of the second peak P2, and take the index of the ECAP sampling data with the largest potential value in the first subset as a second index; then determine a third index according to the second index, and take the potential value of the ECAP sampling data with the largest potential value, among all ECAP sampling data with the index after the third index, as the potential value of the third peak P3.

10. The nerve stimulation apparatus according to claim 9, wherein the control module is further configured to obtain the third index according to a preset index offset and the second index after obtaining the second index, or
the control module is further configured to take all ECAP sampling data with the index after the second index as a third subset, traverse all ECAP sampling data in the third subset according to an index sequence, and subtract the potential value of previous ECAP sampling data from the potential value of each ECAP sampling data in the third subset to obtain a difference, and take the potential value of current ECAP sampling data, at which the difference changes from negative to positive, as the potential value of an immediate trough after the second peak P2, and take the index of the current ECAP sampling data as the third index.

11. The nerve stimulation apparatus according to any one of claims 1 to 10, wherein the control module is further configured to obtain the potential value of the third peak and the potential value of the second peak according to the potential values of the evoked compound action potential obtained in real time after instructing the sensing module to sense the evoked compound action potential.

12. The nerve stimulation apparatus according to any one of claims 1 to 11, wherein the control module includes a first register, a second register, a third register and a counter;
after obtaining a real-time sampled potential value of the evoked compound action potential, the control module compares the real-time sampled potential value of the evoked compound action potential with a first potential value pre-stored in the first register: in a case that the real-time sampled potential value is greater than the first potential value, counting of the counter accumulates; and in a case that the real-time sampled potential value is less than or equal to the first potential value, the real-time sampled potential value is taken as the new first potential value, and the counting of the counter is cleared; when the counting of the counter reaches a first preset threshold, the control module takes the potential value currently stored in the first register as the potential value of a first trough;
after determining the potential value of the first trough, the control module compares the real-time sampled potential value of the evoked compound action potential with a second potential value pre-stored in the second register: in a case that the real-time sampled potential value is less than the second potential value, the counting of the counter accumulates; and in a case that the real-time sampled potential value is greater than or equal to the second potential value, the real-time sampled potential value is taken as the new second potential value; when the counting of the counter reaches a second preset threshold, the control module takes the potential value currently stored in the second register as the potential value of the second peak; and
after determining the potential value of the second peak, the control module compares the real-time sampled potential value of the evoked compound action potential after an immediate trough after the second peak with a third potential value in the third register: in a case that the real-time sampled potential value is less than the third potential value, the counting of the counter accumulates; and in a case that the real-time sampled potential value is greater than or equal to the third potential value, the real-time sampled potential value is taken as the new third potential value, and the counting of the counter is cleared; when the counting of the counter reaches a third preset threshold, the control module takes the potential value currently stored in the third register as the potential value of the third peak.

13. The nerve stimulation apparatus according to claim 12, wherein after obtaining the potential value of the second peak, the control module is further configured to determine whether a real-time offset between the real-time sampled evoked compound action potential and the second peak meets a preset offset threshold, and determines that the evoked compound action potential reaches the immediate trough after the second peak when the real-time offset meets the preset offset threshold.

14. The nerve stimulation apparatus according to claim 12, wherein the control module further includes a fourth register;
after determining the potential value of the second peak, the control module compares the real-time sampled potential value of the evoked compound action potential with a fourth potential value pre-stored in the fourth register: in a case that the real-time sampled potential value is greater than the fourth potential value, the counting of the counter accumulates; and in a case that the real-time sampled potential value is less than or equal to the fourth potential value, the real-time sampled potential value is taken as the new fourth potential value, and the counting of the counter is cleared; when the counting of the counter reaches a fourth preset threshold, the control module takes the potential value currently stored in the fourth register as the potential value of the immediate trough after the second peak.

15. The nerve stimulation apparatus according to any one of claims 1 to 14, wherein the control module is further configured to obtain a peak-to-peak value of ECAP, and to adjust, based on a determination that the peak-to-peak value of ECAP is not within the comfort range, the amplitude of the pulse to be generated within the subsequent pulse generation cycle iteratively until the peak-to-peak value of ECAP is within the comfort range; wherein the comfort range further includes a dimension of peak-to-peak value of ECAP.

16. The nerve stimulation apparatus according to any one of claims 1 to 15, wherein the nerve stimulation apparatus is applied to spinal cord electrical stimulation or dorsal root ganglion electrical stimulation.

17. The nerve stimulation apparatus according to any one of claims 1 to 16, wherein a pulse generation mode of the nerve stimulation apparatus is a tonic bursting mode, and the control module is configured to instruct the sensing module to sense the evoked compound action potential when instructing the pulse generation module to generate a last pulse in a cluster of pulses, to obtain the peak-to-peak ratio of the evoked compound action potential, and determine whether the peak-to-peak ratio of the evoked compound action potential is within the comfort range.

18. A nerve stimulation system, comprising: the nerve stimulation apparatus according to any one of claims 1 to 17 and an electrode lead electrically connected with the nerve stimulation apparatus.

19. An electronic device, comprising:
at least one processor; and
a memory communicatively connected to the at least one processor; wherein
the memory stores instructions executable by the at least one processor, and the instructions, when executed by the at least one processor, cause the at least one processor to implement a method for controlling a nerve stimulation apparatus, and the control method includes:
instructing to sense an evoked compound action potential after a pulse of a current pulse generation cycle is generated;
obtaining a peak-to-peak ratio of the evoked compound action potential, and adjusting, based on a determination that the peak-to-peak ratio of the evoked compound action potential is not within a comfort range, the amplitude of the pulse to be generated in a subsequent pulse generation cycle until the peak-to-peak ratio of the evoked compound action potential is within the comfort range;
wherein the comfort range includes a dimension of amplitude of the pulse and a dimension of peak-to-peak ratio of the evoked compound action potential.

20. A computer-readable storage medium storing a computer program, wherein the computer program, when executed by a processor, causes the processor to implement a method for controlling a nerve stimulation apparatus, and the control method includes:
instructing to sense an evoked compound action potential after a pulse of a current pulse generation cycle is generated;
obtaining a peak-to-peak ratio of the evoked compound action potential, and adjusting, based on a determination that the peak-to-peak ratio of the evoked compound action potential is not within a comfort range, the amplitude of the pulse to be generated in a subsequent pulse generation cycle until the peak-to-peak ratio of the evoked compound action potential is within the comfort range;
wherein the comfort range includes a dimension of amplitude of the pulse and a dimension of peak-to-peak ratio of the evoked compound action potential.
